# EUROPEAN PATENT SPECIFICATION

(11) **EP 1 158 051 B1**
(45) Date of publication and mention of the grant of the patent: **14.12.2005**
(21) Application number: 01112221.5
(22) Date of filing: 18.05.2001
(51) Int. Cl.: C12N 15/53, C12N 15/11, C12N 9/04, C12N 1/15, C12P 23/00

(54) **Process for producing astaxanthin**
Verfahren zur Herstellung von Astaxanthin
Procédé pour la production d' astaxanthin

(30) Priority: 24.05.2000 EP 00111148
(43) Date of publication of application: 28.11.2001
(73) Proprietor: DSM IP Assets B.V., 6411 TE Heerlen (NL)
(72) Inventor: Hoshino, Tatsuo, Kamakura-shi, Kanagawa-ken 248-0027 (JP); Ojima, Kazuyuki, Fujisawa-shi, Kanagawa-ken 251-0877 (JP); Setoguchi, Yutaka, Fujisawa-shi, Kanagawa-ken 251-0033 (JP)
(74) Representative: Schwander, Kuno

(56) References cited:
- EP-A- 0 955 363
- WO-A-00/44920
- WO-A-90/01552
- WO-A-91/02060
- WO-A-94/06918
- WO-A-97/23633
- DATABASE EMBL SEQUENCE DATANASE [Online] Hinxton, UK; 24 January 1996 (1996-01-24) Q. LI ET AL.: "Neurospora crassa alternative oxidase (ALTOX) gene, complete cds." XP002176769 -& LI Q., RITZEL R.G., MCLEAN L.L., MCINTOSH L., KO T., BERTRAND H.,: "Cloning and analysis of the alternative oxidase gene of Neurospora" GENETICS , vol. 142, no. 1, 1996, pages 129-140, XP001024030 Genetic Society, Baltimore, MD, US
- SCHROEDER W A ET AL: "SINGLET OXYGEN AND PEROXYL RADICALS REGULATE CAROTENIOD BIOSYNTHESIS IN PHAFFIA RHODOZYMA" JOURNAL OF BIOLOGICAL CHEMISTRY, AMERICAN SOCIETY OF BIOLOGICAL CHEMISTS, BALTIMORE, MD, US, vol. 270, no. 31, 4 August 1995 (1995-08-04), pages 18374-18379, XP000577110 ISSN: 0021-9258
- E.A. JOHNSON AND W.A SCHROEDER: "Biotechnology of astaxanthin production in Phaffia rhodozyma" BIOTECHNOLOGY FOR IMPROVED FOODS AND FLAVORS, vol. 637, November 1996 (1996-11) - December 1996 (1996-12), pages 39-50, XP001016397 Am. Chem Soc., Washington, us
- AN G-H ET AL: "INFLUENCE OF LIGHT ON GROWTH AND PIGMENTATION OF THE YEAST PHAFFIA-RHODOZYMA" ANTONIE VAN LEEUWENHOEK, vol. 57, no. 4, 1990, pages 191-203, XP001016379 ISSN: 0003-6072
- MARTINEZ C ET AL: "GENETIC TRANSFORMATION OF ASTAXANTHIN MUTANTS OF PHAFFIA RHODOZYMA" ANTONIE VAN LEEUWENHOEK, DORDRECHT, NL, vol. 73, 1998, pages 147-153, XP000993172

## Description

### FIELD OF THE INVENTION

The present invention relates to molecular biology for the manufacture of carotenoids and biological materials useful therefor.

### BACKGROUND OF THE INVENTION

Astaxanthin is known to be distributed in a wide variety of organisms such as animal (e.g. birds such as flamingo and scarlet ibis, and fish such as rainbow trout and salmon), algae and microorganisms. It is also recognized that astaxanthin has a strong antioxidation property against reactive oxygen species, which is expected to apply to pharmaceutical usage to protect living cells against some diseases such as a cancer. Moreover, from a viewpoint of industrial application, a demand for astaxanthin as a coloring reagent is increasing especially in the industry of farmed fish, such as salmon, because astaxanthin imparts distinctive orange-red coloration to the animals and contributes to consumer appeal in the marketplace.

*Phaffia rhodozyma* is known as a carotenogenic yeast strain which produces astaxanthin specifically. Different from the other carotenogenic yeast, *Rhodotorula* species, *Phaffia rhodozyma* (*P. rhodozyma*) can ferment some sugars such as D-glucose. This is an important feature from a viewpoint of industrial application. In a recent taxonomic study, a sexual cycle of *P*. *rhodozyma* was revealed and its telemorphic state was designated under the name of *Xanthophyllomyces dendrorhous* (W.I. Golubev; Yeast 11, 101 - 110, 1995). Some strain improvement studies to obtain hyper producers of astaxanthin from *P*. *rhodozyma* have been conducted, but such efforts have been restricted to employ the method of conventional mutagenesis and protoplast fusion in this decade. Recently, Wery *et al*. developed a host vector system using *P*. *rhodozyma* in which a non-replicable plasmid was used to be integrated onto the genome of *P. rhodozyma* at the locus of ribosomal DNA in multicopies (Wery *et al*., Gene, 184, 89-97, 1997). And Verdoes *et al*. reported more improved vectors to obtain a transformant of *P*. *rhodozyma* as well as its three carotenogenic genes which code the enzymes that catalyzes the reactions from geranylgeranyl pyrophosphate to beta-carotene (International patent publication WO97/23633). The importance of genetic engineering method on the strain improvement study of *P. rhodozyma* will increase in near future to break through the reached productivity by the conventional methods.

Many researchers have speculated that astaxanthin might play a role as an antioxidant in *Phaffia rhodozyma* because its production is stimulated in a respiration phase of growth rather than in the fermentation phase. In general, reactive oxygen species tend to be generated in the respiration phase as a result of electron overflow in the respiratory chain which is caused by the imbalance of the electron transfer between reduction speed of ubiquinone pool and electron transfer in the downstream of the respiratory chain. In such a speculation, astaxanthin might quench such reactive oxygen species as super oxide dismutase does in living organisms.

Schroeder *et al*. reported that respiratory chain of *Phaffia rhodozyma* was shifted from KCN-sensitive respiration to KCN-resistant one in the late phase of growth when astaxanthin production was stimulated (J. Biol. Chem., 270, 18374-18379, 1995). KCN-sensitive respiratory chain is the common electron transfer chain, in which the electron within the ubiquinone pool is transferred to complex IV via complex III which is distributed in a wide variety of organisms. It is known that this respiratory chain is inhibited by KCN or antimycin A. On the other hand, KCN-resistant respiratory chain is distributed in plant and fungi. In this respiratory chain, mitochondrial membrane protein called as alternative oxidase (AOX) plays a substantial role to transfer the electron within the ubiquinone pool to H₂O molecule by using an oxygen molecule as a receptor. AOX activity is known to be inhibited by n-propyl gallate (n-PG) or salicylhydroxamic acid (SHAM).

In their characterization study for antimycin-sensitive hyper producers of astaxanthin derived from *Phaffia rhodozyma*, An *et al*. made a speculation that such mutants produced much more astaxanthin to quench the reactive oxygen species, which might be produced by the electron overflowed from the electron transfer chain (Appl. Env. Microbiol, 55, 116-124, 1989).

"Neurospora crassa alternative oxidase ...", Q Liet et al., Genetics, vol. 142, no. 1, 129-140, 1996 describes the cloning and analysis of the alternative oxidase gene of Neurospora crassa. Mitochondria of Neurospora crassa contain a cyanide-resistant alternative respiratory pathway in addition to the cytochrome pathway. The alternative oxidase is present only when electron flow through the cytochrome chain is restricted. Both genomic anc cDNA copies for the alternative oxidase gene have been isolated and analysed. This publication discloses also the nucleic acid sequence of the Neurospora crassa alternative oxidase (ALTOX) gene respectively the corresponding amino acid coding sequence.

WO 91 02060 describes the production of a yeast having an enhanced astaxanthin content which comprises subjecting the yeast to growth in the presence of a metabolic pathway inhibitor under an influence which triggers a secondary respiratory (oxidative) pathway, or subjecting a Phaffia yeast obtained as above, to growth in the presence of a main respiratory pathway inhibitor and in presence of an agent or under the influence of an environmental condition which triggers a secondary respiratory (oxidative) pathway.

WO 90 01552 describes a process for the production of a yeast having an enhanced astaxanthin (AX) content comprising (a) morphological selection by culturing a yeast of the genus Phaffia in a nutrient medium containing an antibiotic, cytochrome B inhibitor or a terpenoid synthetic pathway inhibitor and (b) before and/or after morphological selection, subjecting the yeast to a total of 2 or more steps of mutagenesis.

WO 91 02060 and WO 90 01552 respectively differ from the subject-matter that they lack the technical feature of producing carotenoids or establishing and selecting a mutant strain capable of producing carotenoids in an enhanced level under the condition for reducing an alternative oxidase activity. This invention is created based on the assumption that biosynthesis of astaxanthin might be upregulated under the condition which the electron transfer chain would be in the reduced state. Such a reduced state might be induced by an addition of a specific inhibitor such as antimycin A, KCN, n-PG or SHAM. Such a state also might be induced by some mutation which would result in an imbalance of electron transfer.

In accordance with this invention, mutants on which the resistance against SHAM was conferred were obtained. Such mutants acquired 50 % higher productivity of astaxanthin than their parent strain.

This invention involves the cloning of a gene, which codes for alternative oxidase from *Phaffia rhodozyma*. This invention also involves the enzymatic characterization as a result of the expression of such a gene in suitable host organisms such as *E*. *coli* or *Saccharomyces cerevisiae*. The gene thus cloned may be used for the reduction in AOX activity by using site-directed mutagenesis of promoter sequence or anti-sense method in a suitable host, such as *P*. *rhodozyma*. And their effects on the carotenogenesis can be confirmed by the cultivation of such a transformant in an appropriate medium under an appropriate cultivation condition.

### SUMMARY OF THE INVENTION

The present invention provides a novel process for producing carotenoids, which comprises cultivating an organism which is obtainable by treating a parent organism producible of carotenoids under the condition to induce a reduction of an alternative oxidase activity and selecting an organism of which productivity of carotenoids is enhanced. The organism utilized in the process of the present invention may be a mutant strain of which productivity of carotenoids is enhanced with the aid of alteration of the resistance against an alternative oxidase inhibitor. The said organism may be a mutant resistant to an alternative oxidase inhibitor. A process according to the present invention can be practiced by using an organism which belongs to the kingdom of Protista or Fungi, more preferably to the genus *Synechococcus*, *Synechocystis*, *Haematococcus*, *Dunaliella, Phaffia, Xanthophyllomyces, Neurospora, Rhodotorula, Blakeslea,* or *Phycomyces,* wherein the most preferable organism may be a strain of *Phaffia rhodozyma* and *Xanthophyllomyces dendrorhous*.

The alternative oxidase inhibitor which may be used for the present invention may be selected from the group consisting of n-propyl gallate and salicylhydroxamic acid.

The present invention also provides a method for establishing a mutant strain capable of producing carotenoids in an enhanced level relative to a parent organism, which comprises cultivating an organism producible of carotenoids under the condition for reducing an alternative oxidase activity and selecting an organism capable of producing carotenoids in the level higher than the said parent organism. The said condition for reducing an alternative oxidase activity may comprise the presence of an alternative oxidase inhibitor. The alternative oxidase inhibitor for this purpose may be selected from the group consisting of n-propyl gallate and salicylhydroxamic acid. The organism for a mutant strain of the present invention may belong to the kingdom of Protista or Fungi, more preferably to the genus *Synechococcus*, *Synechocystis, Haematococcus, Dunaliella*, *Phaffia, Xanthophyllomyces, Neurospora, Rhodotorula*, *Blakeslea*, or *Phycomyces,* wherein the most preferable organism may be a strain of *Phaffia rhodozyma* and *Xanthophyllomyces dendrorhous.*

In another aspect, the present invention also relates to a mutant strain of an organism capable of producing carotenoids in enhanced level relative to a parent organism which is obtainable by the method described above. The said mutants may be more specifically characterized in that they can grow even in the medium containing 0.3 to 0.45 mg/ml of SHAM in a similar growth rate in the medium which does not contain SHAM.

As an embodiment of the present invention, there are provided SHAM-resistant mutants, which were derived from *Phaffia rhodozyma* ATCC96594. Such SHAM - resistant mutants were deposited at the DSMZ (Deutsche Sammlung der Mikroorganismen und Zellkulturen GmbH, Mascheroder Weg 1b, D-38124 Braunschweig, Germany) under DSM 13429, DSM 13430 und DSM 13431, respectively, on April 3, 2000.

The organism used in the process of the present invention may be a recombinant organism of which gene expression of the alternative oxidase is altered to reduce efficiency compared to a parent organism. The present invention further provides a recombinant organism capable of producing carotenoids in an enhanced level relative to a host organism which is characterized in that whose gene expression of the alternative oxidase is altered to reduce efficiency compared to the host organism. Such an organism may be that the gene expression of the alternative oxidase is altered with the aid of the technique selected from antisense technology, site-directed mutagenesis, chemical mutagenesis, etc. The organism for this purpose may belong to the kingdom of Protista or Fungi, more preferably to the genus *Synechococcus*, *Synechocystis*, *Haematococcus*, *Dunaliella*, *Phaffia*, *Xanthophyllomyces*, *Neurospora*, *Rhodotorula*, *Blakeslea*, or *Phycomyces,* wherein the most preferable organism may be a strain of *Phaffia rhodozyma* and *Xanthophyllomyces dendrorhous -* especially the above mentioned deposits.

The present invention also provides a recombinant DNA sequence coding for an alternative oxidase derived from an organism producible of carotenoids. The recombinant DNA can be obtained from an organism which belongs to the kingdom of Protista or Fungi, more preferably to the genus *Synechococcus*, *Synechocystis*, *Haematococcus*, *Dunaliella*, *Phaffia*, *Xanthophyllomyces*, *Neurospora*, *Rhodotorula*, *Blakeslea*, or *Phycomyces,* wherein the most preferable organism may be a strain of *Phaffia rhodozyma* and *Xanthophyllomyces dendrorhous -* especially the above mentioned deposits. The recombinant DNA sequence according to the present invention may be that identified by SEQ ID NO: 2 or that having identity with SEQ ID NO: 2 higher than 55%, more preferably higher than 75%, most preferably higher than 95%.

The said recombinant DNA sequence may be more specifically characterized in that (a) it codes for the said enzyme having an amino acid sequence described in SEQ ID NO: 1, or (b) it codes for a variant of the said enzyme selected from (i) an allelic variant, and (ii) an enzyme having one or more amino acid addition, insertion, deletion and/or substitution and having the stated enzyme activity. Particularly specified isolated DNA sequence mentioned above may be that which can be derived from a gene of *Phaffia rhodozyma* and is selected from (i) a DNA sequence represented in SEQ ID NO: 2, (ii) an isocoding or an allelic variant for the DNA sequence represented in SEQ ID NO: 2, and (iii) a derivative of a DNA sequence represented in SEQ ID NO: 2, with addition, insertion, deletion and/or substitution of one or more nucleotide(s), and coding for a polypeptide having the said enzyme activity.

The present invention also provides the use of the said recombinant DNA to transform a host organism. A convenient form of the recombinant DNA may be a vector. The recombinant organism obtained by use of the recombinant DNA is capable of decreasing an enzyme activity for alternative oxidase. The host organism transformed with the recombinant DNA may be useful in the improvement of the production process of carotenoids, in particular astaxanthin. Thus the present invention also provides such a recombinant organism.

Moreover, the present invention provides a method for the biological production of carotenoids which comprises introducing a recombinant DNA described above into an appropriate host organism and cultivating thus obtained recombinant organism. Thus the method for producing a carotenoid, characterized in that a recombinant organism described above is cultivated under conditions conductive to the production of the carotenoid is one of the aspects of the present invention. This method may be applied to the biological production of astaxanthin.

### BRIEF EXPLANATION OF DRAWING

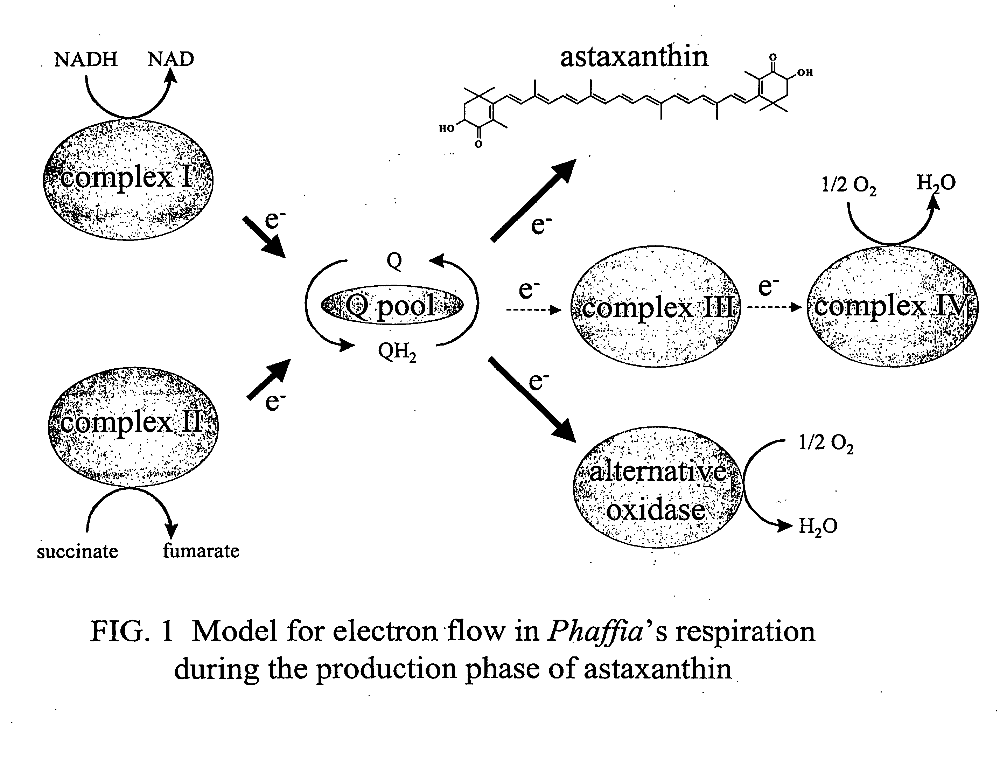

Figure 1 depicts a working model for respiratory chain in *P. rhodozyma*.

### DETAILED DESCRIPTION OF THE INVENTION

As described above, many researchers have speculated that astaxanthin might play a role as an antioxidant in *Phaffia rhodozyma* because its production is stimulated in a respiration phase of growth rather than in the fermentation phase. In general, reactive oxygen species tends to be produced in the respiration phase as a result of electron overflow in the respiratory chain which is caused by the imbalance of the electron transfer between reduction speed of ubiquinone pool and electron transfer in the downstream of the respiratory chain (FIG. 1). In such a speculation, astaxanthin might quench such reactive oxygen species as super oxide dismutase does.

Based on this speculation, overproduction of astaxanthin could be realized by the inhibition of respiratory chain in *Phaffia rhodozyma*. In fact, An *et al*. isolated mutants whose KCN-sensitive respiration were blocked and which produced much more astaxanthin from *Phaffia rhodozyma* (Appl. Env. Microbiol, 55, 116-124, 1989).

On the other hand, as Schroeder *et al*. reported, respiratory chain of *Phaffia rhodozyma* was shifted from KCN-sensitive respiration to KCN-resistant one in the late phase of growth when astaxanthin production was stimulated (J. Biol. Chem., 270, 18374-18379, 1995). In this context, KCN-resistant respiration, which is mediated by alternative oxidase would give more effect on the respiration in the production phase of astaxanthin. The inhibition of alternative oxidase might lead to the overproduction of astaxanthin.

In order to examine the effect of specific inhibition of respiration activity on production of astaxanthin in *Phaffia rhodozyma*, SHAM, which is known to inhibit the alternative oxidase was added to growing agar medium at serial diluted concentration. In the course of this study, several spontaneous mutants, which showed similar growth activity even in the presence of 0.3 to 0.45 mg/ml SHAM to that in the medium which did not include SHAM appeared. Surprisingly it was found that such mutants produced 50 % higher astaxanthin than their parent. This indicated that some mutation which led to overproduction of astaxanthin could complement growth inhibition by the reduced state of respiratory chain which would be caused by a decrease of alternative oxidase activity.

The present invention provides thus isolated mutants which are resistant against 0.3 to 0.45 mg/ml of the specific inhibitor for alternative oxidase, SHAM. Likewise the above, as it is readily understood by those ordinary skilled in the art, mutant strains which are capable of producing astaxanthin in higher productivity can be established by cultivation of an appropriate organisms in the presence of any one or more of the inhibitors to the alternative oxidase, and by screening the organisms showing growth activity in the presence of the said inhibitor(s) and higher productivity of astaxanthin than parent organisms. Productivity of astaxanthin can be determined by extracting carotenoids from the cells of *P*. *rhodozyma* and measuring astaxanthin level as exemplified in Example 2. An increase of the productivity in about 10 % will be a possible criteria to select a mutant strain capable of producing astaxanthin in higher level than a parent strain. Re-cultivation and screening of thus obtained mutant strain under the pressure of the alternative oxidase inhibitor can be employed to improve the productivity. Thus obtained mutant strain can be applied to astaxanthin production in an appropriate medium.

In order to decrease the activity for alternative oxidase, an approach employed by genetic engineering has several advantages against an approach by the addition of specific inhibitor such as SHAM to the culture medium. One of those advantages is an economic reason. Addition of such an inhibitor would increase the production cost. And addition of such an inhibitor to the culture medium has another disadvantage in the purification step to remove the added inhibitors from the final product.

Further, the present invention provides an isolated recombinant DNA sequence which codes for alternative oxidase from *Phaffia rhodozyma*.

The said DNA of the present invention can mean a cDNA which contains only open reading frame flanked between the short fragments in its 5'- and 3'- untranslated region, and also a genomic DNA which contains its introns and regulatory sequences such as its promoter and terminator which are involved in the expression of the gene of interest.

At first, we cloned a partial gene fragment containing a portion of *AOX* gene by using degenerate PCR method. The said degenerate PCR is a method to clone a gene of interest which has high homology of amino acid sequence to the known enzyme from other species which has a same or similar function. Degenerate primer, which is used as a primer in degenerate PCR, was designed by a reverse translation of the amino acid sequence to corresponding nucleotides ("degenerated"). In such a degenerate primer, a mixed primer which consists any of A, C, G or T, or a primer containing inosine at an ambiguity code is generally used. In this invention, the mixed primers were used for degenerate primers to clone above gene. PCR condition used is varied depending on primers and gene to clone as described hereinafter.

An entire gene containing its coding region with its intron as well as its regulatory region such as a promoter or terminator can be cloned from a chromosome by screening of genomic library which is constructed in phage vector or plasmid vector in an appropriate host, by using a partial DNA fragment obtained by degenerate PCR as described above as a probe after it was labeled. Generally, *E*. *coli* as a host strain and *E. coli* vector, a phage vector such as lambda phage vector, or a plasmid vector such as pUC vector is often used in the construction of library and a following genetic manipulation such as a sequencing, a restriction digestion, a ligation and the like. In this invention, an *EcoRI* genomic library of *P. rhodozyma* was constructed in the derivatives of lambda vector, lambda gt11. An insert size, what length of insert must be cloned, was determined by the Southern blot hybridization before a construction of a library. In this invention, a DNA which was used for a probe was labeled with digoxigenin (DIG), a steroid hapten instead of conventional ³²P label, following the protocol which was prepared by the supplier (Boehringer-Mannheim, Mannheim, Germany). A genomic library constructed from the chromosome of *P. rhodozyma* was screened by using a DIG-labeled DNA fragment which had a portion of a gene of interest as a probe. Hybridized plaques were picked up and used for further study. After the isolation of positive plaque, insert fragment was subcloned into appropriate plasmid vector which can be conveniently used for sequencing. In this invention, the insert fragment in the positive phage vector was subcloned into pOCUS-2 vector, which was used for construction of transposon-inserted sequencing derivatives (Locus Pocus System, Novagene, Madison, U.S.A.).

In this invention, the automated fluorescent DNA sequencer, ALFred system (Pharmacia, Uppsala, Sweden) was used with an autocycle sequencing protocol in which the Taq DNA polymerase is employed in most cases of sequencing.

After the determination of the genomic sequence, a sequence of a coding region was used for a cloning of cDNA of corresponding gene. The PCR method was also exploited to clone cDNA fragment. The PCR primers whose sequences were identical to the sequence at the 5'- and 3'- end of the open reading frame (ORF) were synthesized with an addition of an appropriate restriction site, and PCR was performed by using those PCR primers. In this invention, a cDNA pool was used as a template in this PCR cloning of cDNA. The said cDNA pool consists of various cDNA species which were synthesized *in vitro* by the viral reverse transcriptase and Taq polymerase (CapFinder Kit manufactured by Clontech, Palo Alto, U.S.A.) by using the mRNA obtained from *P*. *rhodozyma* as a template, cDNA of interest thus obtained can be confirmed in its sequence. Furthermore, cDNA thus obtained can be used for a confirmation of its enzyme activity after the cloning of the cDNA fragment into an expression vector which functions in *E*. *coli* or *S*. *cerevisiae* under the appropriate promoter.

To express a gene which was derived from Eukaryote, a procedure in which cDNA is cloned into an expression vector in *E*. *coli* or *S*. *cerevisiae* is often used. This is caused from a fact that a specificity of intron structure varies among the organisms and an inability to recognize the intron sequence from other species. In fact, Prokaryote has no intron structure in its own genetic background. Even in the yeast, genetic background is different between Ascomycetes to which *Saccharomyces cerevisiae* belongs and Basidiomycetes to which *P*. *rhodozyma* belongs. Wery *et al*. showed that the intron structure of actin gene from *P. rhodozyma* cannot be recognized nor spliced by the ascomycetous yeast, *Saccharomyces cerevisiae* (Yeast, 12, 641-651, 1996).

Some other researchers reported that intron structures of some kinds of the genes involve regulation of their gene expressions (Dabeva, M. D. *et al*., Proc. Natl. Acad. Sci. U.S.A., 83, 5854, 1986). It might be important to use a genomic fragment which has its introns in a case of self-cloning of the gene of an interest whose intron structure involves such a regulation of its own gene expression.

To apply a genetic engineering method for a strain improvement study, it is necessary to study its genetic mechanism in the event such as transcription and translation. It is important to determine a genetic sequence for its upstream activation sequence (UAS), promoter, intron structure and terminator, not only for its exon, to study the genetic mechanism.

According to this invention, the gene which codes for the alternative oxidase was cloned from genomic DNA of *P*. *rhodozyma*, and their genomic sequence containing alternative oxidase (*AOX*) gene including its 5'- and 3'-adjacent regions as well as its intron structures were determined.

Succeeding to the confirmation of the enzyme activity, gene modification study to decrease the alternative oxidase activity can be employed.

In the present invention, the polypeptide sequence includes SEQ ID NO: 2 and fragments thereof having *AOX* activity and polypeptide sequences which hybridize to SEQ ID NO: 2 under stringency conditions which are sufficient to identify specific binding to SEQ ID NO: 2, and which hybrids encode a polypeptide that has the function of an alternative oxidase. For example, any combination of the following hybridization and wash conditions may be used to achieve the required specific binding:

### High Stringency Hybridization:

6X SSC
0.5% SDS
100 micrograms/ml denatured salmon sperm DNA
50% formamide
Incubate overnight with gentle rocking at 42°C overnight.

### High Stringency Wash:

1 wash in 2X SSC, 0.5% SDS at Room Temperature for 15 minutes, followed by another wash in 0.1X SSC, 0.5% SDS at Room Temperature for 15 minutes.

### Low Stringency Hybridization:

6X SSC
0.5% SDS
100 micrograms/ml denatured salmon sperm DNA
50% formamide
Incubate overnight with gentle rocking at 37°C overnight.

### Low Stringency Wash:

1 wash in 0.1X SSC, 0.5% SDS at Room Temperature for 15 minutes.

Moderately stringent conditions may be obtained by varying the temperature at which the hybridization reaction occurs and/or the wash conditions as set forth above. In the present invention, it is preferred to use high stringency hybridization and wash conditions.

To decrease a gene expression with genetic methods, some methods can be exploited. One of such methods is anti-sense method. Anti-sense method is a method to decrease an expression of gene of interest by introducing an artificial gene fragment, whose sequence is complementary to that of gene of interest. And such an anti-sense gene fragment would form complex with mature mRNA fragment of objective gene *in vivo* and inhibit an efficient translation from mRNA, as a consequence. For construction of anti-sense RNA for *AOX* gene, a PCR method can be employed to clone the complementary cDNA strand for *AOX* gene.

The other method is a mutation of promoter region. In general, the gene consists of several parts which have different functions from each other. In Eukaryotes, genes which encode corresponding proteins are transcribed to premature messenger RNA (pre-mRNA) differing from the genes for ribosomal RNA (rRNA), small nuclear RNA (snRNA) and transfer RNA (tRNA). Although RNA polymerase II (PolII) plays a central role in this transcription event, PolII can not solely start transcription without *cis* element covering an upstream region containing a promoter and an UAS, and a *trans*-acting protein factor. At first, a transcription initiation complex which consists of several basic protein components recognizes the promoter sequence in the 5'-adjacent region of the gene to be expressed. In this event, some additional participants are required in the case of the gene which is expressed under some specific regulation, such as a heat shock response, or adaptation to a nutrition starvation, and so on. In such a case, a UAS is required to exist in the 5'-untranslated upstream region around the promoter sequence, and some positive or negative regulator proteins recognize and bind to the UAS. The strength of the binding of transcription initiation complex to the promoter sequence is affected by such a binding of the trans-acting factor around the promoter, and this enables the regulation of the transcription activity.

After the activation of a transcription initiation complex by the phosphorylation, a transcription initiation complex initiates transcription from the transcription start site. Some parts of the transcription initiation complex are detached as an elongation complex from the promoter region to the 3'-direction of the gene (this step is called as a promoter clearance event) and an elongation complex continues the transcription until it reaches to a termination sequence that is located in the 3'-adjacent downstream region of the gene.

To decrease an expression of gene of interest, mutation by conventional chemical mutagenesis or genetical site-directed mutagenesis in the promoter region of objective gene containing UAS sequence described above was often used. In this approach, a gene cassette, containing a reporter gene which is fused to a promoter region derived from a gene of interest at its 5'-end and a terminator region from a gene of interest at its 3'-end, is mutagenized and then introduced into *P. rhodozyma*. By detecting the difference of the activity of the reporter gene, an effective mutation would be screened. Mutant strain in which expression of enzyme of interest might decrease can be obtained by transforming host strain with a recombinant DNA having such a mutated promoter region.

Such constructs as a vector containing anti-sense *AOX* gene or mutated promoter for *AOX* gene can be transferred into appropriate host strain. In the case of using *Phaffia rhodozyma* as a host strain, this is realized by the cloning of such constructs into the appropriate vector on which a selectable marker that functions in *P. rhodozyma* is harbored. A drug resistance gene which encodes the enzyme that enables the host to survive in the presence of a toxic antibiotic is often used for the selectable marker. G418 resistance gene harbored in pGB-Ph9 (Wery *et al*., Gene, 184, 89-97, 1997) is an example of a drug resistance gene. Such a plasmid can be integrated on the chromosome of *Phaffia rhodozyma* through the homologous recombination between the chromosome and the plasmid.

As a transformation method, LiAc method and electroporation method (Wery *et al*., *Gene,* 184, 89-97, 1997) were applied to transform *P. rhodozyma*.

Such a genetically engineered *P. rhodozyma* would be cultivated in an appropriate medium and evaluated in its productivity of astaxanthin.

The present invention is further illustrated with Examples described below by referring to the attached drawing.

### Examples

The following materials and methods were employed in the Examples described below:

### Strains

*P*. *rhodozyma* ATCC96594 (re-deposited under the accession No. ATCC 74438 on April 8, 1998 pursuant to the Budapest Treaty)
*E. coli* Y1090r⁻: araD139, *hsd*R (r_{K}⁻, m_{K}⁺), *mcr*B^{*+*}*, rps*L*, sup*F*,* trpC12::Tn10, Δ*lac*U169, Δ*lon,* F⁻, λ⁻, (pMC9) (Clontech)
*E. coli* DH5alpha: F⁻, φ80d, *lac*ZΔM15, Δ(*lac*ZYA*-arg*F)U169*, hsd* (r_{K}⁻, m_{K}⁺), *rec*A1*, end*A1*, deo*R*, thi-1, gyr*A96*, rel*A1 (Toyobo, Osaka, Japan)
*E. coli* gamma delta donor: Δ(*gpt-pro*A)62*, leu,* 44, *ara*14, *gal*K2*,* lacY1, Δ(*mcrC-mrr*)*,* (r_{B}⁻, m_{B}⁻), *xyl-*5*, mt*l*-*1*, rec*A13, [F⁺ :: Tn1000 (tet^{s})] (Novagene)
*E. coli* gamma delta recipient: F⁻, araD139, Δ(*ara-leu*)7696*, gal*E15*, gal*K16, Δ(lac)X74, (Str^{r}), *hsd*R2 (r_{K12}⁻, m_{K12}⁺), *mcr*A*,mcr*B1::Tn5 (kan^{r}) (Novagene)
*E. coli* TOP10: F⁻, *mcr*A*,* Δ(*mrr-hsd*RMS-*mcr*BC)*,* φ80, M15, Δ*lac*X74*, rec*A1*, deo*R*, ara*D139*,* (*ara-leu*)7697*, gal*U*, gal*K*, rps*L (Str^{r}), *end*A1, *nup*G (Invitrogen, NV Leek, the Netherlands)

### Vectors

lambda gt11 (Clontech)
pCR2.1-TOPO (Invitrogen)
pOCUS-2
pBluescript II SK-(Stratagene)
pGBPh9 (Wery et al., Yeast, 12, 641-651, 1996)

### Media

*P*. *rhodozyma* strain is maintained routinely in YPD medium (DIFCO, Detroit, U.S.A.). *E*. *coli* strain is maintained in LB medium (10 g Bacto-trypton (DIFCO), 5 g yeast extract (DIFCO) and 5 g NaCl per liter). NZY medium (5 g NaCl, 2 g MgSO₄-7H₂O, 5 g yeast extract (DIFCO), 10 g NZ amine type A (WAKO, Osaka, Japan) per liter) is used for lambda phage propagation in a soft agar (0.7 % agar; WAKO). When an agar medium was prepared, 1.5 % of agar (WAKO) was supplemented. Salicylhydroxamic acid (SHAM) was purchased from Aldrich (Milwaukee, U.S.A.).

### Methods

A general method for techniques of molecular genetics was according to the method in Molecular cloning: a Laboratory Manual, 2nd Edition (Cold Spring Harbor Laboratory Press, 1989). Restriction enzymes and T4 DNA ligase were purchased from Takara Shuzo (Ohtsu, Japan).

Isolation of a chromosomal DNA from *P. rhodozyma* was performed by using QIAGEN Genomic Kit (QIAGEN, Hilden, Germany) following the protocol supplied by the manufacturer. Mini-prep of plasmid DNA from transformed *E*. *coli* was performed with the Automatic DNA isolation system (PI-50, Kurabo, Co. Ltd., Osaka, Japan). Midi-prep of plasmid DNA from an *E*. *coli* transformant was performed by using QIAGEN column (QIAGEN). A DNA fragment was isolated and purified from agarose by using QIAquick or QIAEX II (QIAGEN).

Isolation of total RNA from *P. rhodozyma* was performed with phenol method by using Isogen (Nippon Gene, Toyama, Japan), mRNA was purified from total RNA thus obtained by using mRNA separation kit (Clontech). cDNA was synthesized by using CapFinder cDNA construction kit (Clontech).

*In vitro* packaging was performed by using Gigapack III gold packaging extract (Stratagene, La Jolla, U.S.A.). Isolation of lambda DNA was performed by Wizard lambda preps DNA purification system (Promega, Madison, U.S.A.) following the protocol prepared by the manufacturer.

Polymerase chain reaction (PCR) was performed with the thermal cycler from Perkin Elmer model 2400. Each PCR condition is described in the Examples. PCR primers were purchased from a commercial supplier. DNA sequencing was performed with the automated fluorescent DNA sequencer (ALFred, Pharmacia).

Competent cells of DH5alpha were purchased from Toyobo. All the chemicals were purchased from WAKO otherwise stated.

### Example 1 Isolation of SHAM-resistant mutants, SHAM1, SHAM2 and SHAM3 from P. rhodozyma ATCC96594

In order to examine the effect of the inhibition for KCN-resistant respiration mediated by alternative oxidase on the growth of *P*. *rhodozyma*, addition of SHAM to culture of *P*. *rhodozyma* on YPD-agar medium was conducted. SHAM was resolved in ethanol and added to the YPD-agar medium by 0.05, 0.15, 0.30, 0.45 and 0.90 mg/ml at final concentration, respectively. On those medium, 2 x 10⁷ cells / ml of *P. rhodozyma* ATCC96594 were spread after dilution. After 3 day-cultivation at 20 °C, yielded colonies were counted. As a result, the number of colonies which grew on SHAM-containing YPD agar was almost the same as that on control medium which did not contain SHAM. But, size of colonies which grew on SHAM-containing medium was smaller than control culture. TABLE 1 shows the relative colony diameter against the control colony.

**TABLE 1**

| Relative size of colony which grew on SHAM-containing YPD-agar | | | | | |
|---|---|---|---|---|---|
| SHAM (mg/ml) | 0.05 | 0.15 | 0.30 | 0.45 | 0.90 |
| Relative colony diameter (%) | 100 | 70 | 40 | 30 | 12 |

Interestingly, among the colonies, which grew on the medium containing 0.3 and 0.45 mg/ml of SHAM, some colonies showed the similar size to the control colony. Such colonies also showed deeper pigmentation than the control colony. Four colonies, which showed similar colony size to the control colony were picked up and streaked onto YPD-agar medium. All the colonies showed deeper pigmentation than the control colony even on YPD-agar medium, which did not contain SHAM. From this result, it was suggested that these strains might be spontaneous mutants and were named as SHAM1, SHAM2, SHAM3 and SHAM4.

### Example 2 Flask fermentation of resistant mutants, SHAM1, SHAM2 and SHAM3

To evaluate productivity of astaxanthin by SHAM-resistant mutants, SHAM1, SHAM2 and SHAM3, fermentation in shaking flasks was conducted. These mutants and their parent strain, ATCC96594 were inoculated from freshly prepared agar-culture to 50 ml of YPD medium in baffle flask of 500 ml in size at final OD at 660 nm of 0.05. Fermentation was conducted at 20 °C at 200 r.p.m. At an appropriate interval, 3 ml of broth was withdrawn and was analyzed for cell yield and astaxanthin content.

Cell yield was measured as OD at 660 nm. As dry cell weight, it was measured by weighing cells derived from 1.0 ml of broth after heating at 120 °C overnight in 1.5 ml of microcentrifugation tube. Astaxanthin content of *P. rhodozyma* was measured with HPLC method after extraction of carotenoids from cells of *P*. *rhodozyma* by disruption with glass beads as follows. Cells obtained from 1 ml of broth after the centrifugation was concentrated by two-fold with distilled water and 10.0 gram of glass beads was added to the cell suspension (0.5 ml) in brown-shaded test tube (13.5 mm, 11 cm). Next, 1.5 ml of acetone / butylated hydroxy toluene (BHT) /water (45 mg of BHT in 450 ml acetone and 50 ml water) was added and then test tube was shaken with horizontal table top shaker for an hour. After extraction, 5 ml of acetone/BHT/water containing appropriate concentration of bixin (nacalai tesque, Kyoto, Japan) as an internal standard was added. Supernatant was analyzed for astaxanthin content with following HPLC system. (Hardware for HPLC system was purchased from Tosoh (Tokyo, Japan)).
HPLC column; YMC-Pak ODS-A (6 mm, 150 mm (YMC, Inc., Milford, U.S.A.).
Temperature; room temperature
Eluent; acetonitrile / methanol / isopropanol (85 / 10 / 5)
Injection volume; 10 microliter
Flow Rate; 2.0 ml/minute
Detection; UV at 471 nm

Results are summarized in TABLE 2. All the mutants showed 50 % higher productivity of astaxanthin than parent strain, ATCC96594. From this result, some mutation might occur in these mutants to compensate the inhibition of alternative oxidase activity by increase of astaxanthin production.

**TABLE 2**

| Productivity of astaxanthin by SHAM-resistant mutants astaxanthin productivity | | | | | | |
|---|---|---|---|---|---|---|
| (mg/L) | (mg/g-dried cell) | | OD@660nm | | | |
| (hours) | 38 | 72 | 38 | 72 | 38 | 72 |
| SHAM-1 | 1.65 | 4.07 | 0.179 | 0.380 | 24.5 | 30.6 |
| SHAM-2 | 2.36 | 4.43 | 0.217 | 0.385 | 29.5 | 30.5 |
| SHAM-3 | 2.87 | 3.97 | 0.247 | 0.381 | 29.2 | 29.5 |
| ATCC96594 | 2.00 | 2.76 | 0.164 | 0.258 | 27.4 | 28.7 |

### Example 3 Isolation of mRNA from P. rhodozyma and construction of cDNA library

To construct cDNA library of *P*. *rhodozyma*, total RNA was isolated by phenol extraction method right after the cell disruption and the mRNA from *P. rhodozyma* ATCC96594 strain was purified by using mRNA separation kit (Clontech).

At first, cells of ATCC96594 strain from 10 ml of two-day-culture in YPD medium were harvested by centrifugation (1500 x g for 10 min.) and washed once with extraction buffer (10 mM Na-citrate / HCl (pH 6.2) containing 0.7 M KCl). After suspending in 2.5 ml of extraction buffer, the cells were disrupted by French press homogenizer (Ohtake Works Corp., Tokyo, Japan) at 1500 kgf/cm² and immediately mixed with two times of volume of isogen (Nippon gene) according to the method specified by the manufacturer. In this step, 400 microgram of total RNA was recovered.

Then this total RNA was purified by using mRNA separation kit (Clontech) according to the method specified by the manufacturer. Finally, 16 gram of mRNA from *P. rhodozyma* ATCC96594 strain was obtained.

To construct cDNA library, CapFinder PCR cDNA construction kit (Clontech) was used according to the method specified by the manufacturer. One microgram of purified mRNA was applied for a first strand synthesis followed by PCR amplification. After this amplification by PCR, 1 mg of cDNA pool was obtained.

### Example 4 Cloning of the partial AOX (alternative oxidase) gene from P. rhodozyma

To clone a partial *AOX* gene from *P. rhodozyma*, a degenerate PCR method was exploited. Species and accession number to database whose sequence for alternative oxidase were used for multiple alignment analysis (ClustalW, Thompson J. D., et al., Nucleic Acids Research, 22, 4673-4680, 1994) are as follows.

| | |
|---|---|
| *Aspergillus niger* | AB016540 (DDBJ/GenBank/EMBL) |
| *Candida albicans* | AF031229 (DDBJ/GenBank/EMBL) |
| *Chlamydomonas reinhardtii* | AF047832 (DDBJ/GenBank/EMBL) |
| *Magnaporthe grisea* | AB005144 (DDBJ/GenBank/EMBL) |
| *Neurospora crassa* | Q01355 (Swissprot) |
| *Oryza sativa* | AB004813 (DDBJ/GenBank/EMBL) |
| *Pichia anomala* | Q00912 (Swissprot) |
| *Trypanosoma brucei brucei* | Q26710 (Swissprot) |

Two mixed primers whose nucleotide sequences were designed and synthesized as shown in TABLE 3 based on the common sequence of known alternative oxidase genes from other species.

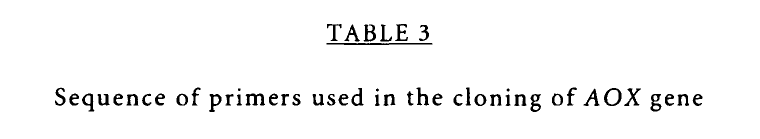

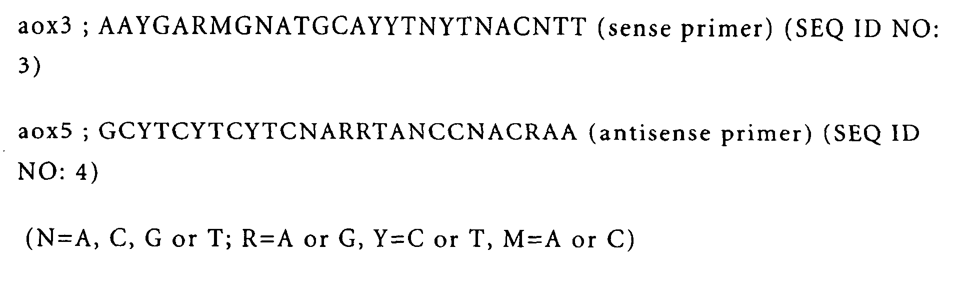

After the PCR reaction of 25 cycles of 95 °C for 30 seconds, 50 °C for 30 seconds and 72°C for 15 seconds by using ExTaq (Takara Shuzo) as a DNA polymerase and cDNA pool obtained in Example 1 as a template, reaction mixture was applied to agarose gel electrophoresis. A PCR band that had a desired length was recovered and purified by QIAquick (QIAGEN) according to the method by the manufacturer and then ligated to pCR2.1-TOPO (Invitrogen). After the transformation of competent *E*. *coli* TOP10, 6 white colonies were selected and plasmids were isolated with Automatic DNA isolation system. As a result of sequencing, it was found that 3 clones had a sequence whose deduced amino acid sequence was similar to known alternative oxidase genes. This isolated cDNA clone was designated as pAOX514 and used for further study.

### Example 5 Isolation of genomic DNA from P. rhodozyma

To isolate a genomic DNA from *P*. *rhodozyma*, QIAGEN genomic kit was used according to the method specified by the manufacturer.

At first, cells of *P*. *rhodozyma* ATCC96594 strain from 100 ml of overnight culture in YPD medium were harvested by centrifugation (1500 x g for 10 min.) and washed once with TE buffer (10 mM Tris / HCl (pH 8.0) containing 1 mM EDTA). After suspending in 8 ml of Y1 buffer of the QIAGEN genomic kit, lyticase (SIGMA, St. Louis, U.S.A.) was added at the concentration of 2 mg/ml to disrupt cells by enzymatic degradation and the reaction mixture was incubated for 90 minutes at 30 °C and then proceeded to the next extraction step. Finally, 20 microgram of genomic DNA was obtained.

### Example 6 Southern blot hybridization by using pAOX514 as a probe

Southern blot hybridization was performed to clone a genomic fragment which contains *AOX* gene from *P. rhodozyma*. Two microgram of genomic DNA was digested by *EcoRI* and subjected to agarose gel electrophoresis followed by acidic and alkaline treatment. The denatured DNA was transferred to nylon membrane (Hybond N+, Amersham, Buckinghamshire, U.K.) by using transblot (Joto Rika, Tokyo, Japan) for an hour. The DNA which was transferred to nylon membrane was fixed by a heat treatment (80 °C, 90 minutes). A probe was prepared by labeling a template DNA (*Eco*RI-digested pAOX514) with DIG multipriming method (Boehringer Mannheim). Hybridization was performed with the method specified by the manufacturer. As a result, hybridized band was visualized in the range from 5.5 to 7.0 kilobases (kb).

### Example 7 Cloning of a genomic fragment containing AOX gene

Four microgram of the genomic DNA was digested by *EcoRI* and subjected to agarose gel electrophoresis. Then, DNAs whose length is within the range from 5.0 to 7.0 kb was recovered by QIAEX II gel extraction kit (QIAGEN) according to the method specified by the manufacturer. The purified DNA was ligated to 0.5 microgram of *Eco*RI-digested and CIAP (calf intestine alkaline phosphatase)-treated lambda gt11 (Clontech) at 16 °C overnight, and packaged by Gigapack III gold packaging extract (Stratagene). The packaged extract was infected to *E*. *coli* Y1090 strain and over-laid with NZY medium poured onto LB agar medium. About 6000 plaques were screened by using EcoRI-digested pAOX514 as a probe. One plaque was hybridized to the labeled probe.

This lambda gt11 derivative containing putative *AOX* gene from *P*. *rhodozyma* was prepared by using Wizard lambda preps DNA purification system (Promega). As a result of digestion by *EcoR*I*,* it was revealed that this lambda gt11 derivative contained 6 kb *EcoRI* insert. Next, PCR was conducted by using this lambda gt11 derivative as a template and two primers, aox3 and aox5 as primers. As a result of PCR under the same PCR condition as described in Example 4, expected 0.3 kb band was yielded. It was suggested that this lambda gt11 derivative might contain putative *AOX* gene from *P*. *rhodozyma*. 6.0 kb insert EcoRI fragment in this lambda gt11 derivative was purified by using QIAquick (QIAGEN) and subjected to subcloning into pOCUS-2 vector (Novagen) by using DH5alpha as a host strain and yielded pOCUSAOX607.

### Example 8 Sequencing of a genomic fragment containing AOX gene

pOCUSAOX607 was transferred into competent gamma delta donor cells and used for preparation of sequencing derivatives which were used for Locus Pocus system (Novagen). Method for preparation of sequencing derivatives was followed by the protocol supplied by the manufacturer. As sequencing primers, Cy5-labelled primers whose sequence are listed in TABLE 4 were synthesized and used for sequencing by using AutoCycle sequencing kit (Pharmacia).

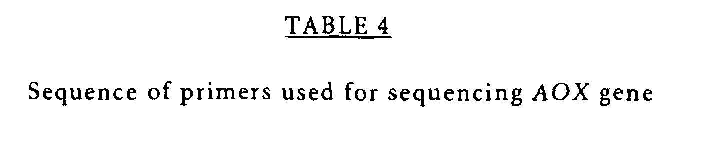

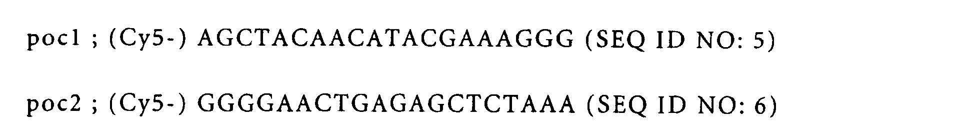

As a result of sequencing, nucleotide sequence comprising 2561 base pairs of genomic fragment containing *AOX* gene from *P. rhodozyma* was determined.

Coding region was in 1206 base pairs that consisted of 10 exons and 9 introns. Introns were dispersed all through the coding region without 5' or 3' bias. By using genetic analysis software, GENETYX-SV/RC (Software Development Co., Ltd., Tokyo, Japan) version 4.0.1, it was found that open reading frame consists of 402 amino acids (SEQ ID NO: 1) whose sequence is strikingly similar to the known amino acid sequence of alternative oxidase from other species (51.5 % identity to alternative oxidase from *Aspergillus niger*). A stretch of hydrophobic amino acid residues at amino terminal end which was expected to form alpha-helix structure indicated that this amino terminal region might be a membrane spanning domain or transit peptide for mitochondria. PSORTII program (http://psort.nibb.ac.jp:8800/) predicted that this protein might be mitochondrial protein by 82.6 % prediction value.

### Example 9 Cloning of upstream region of AOX gene

Cloning of 5'- adjacent region of *AOX* gene was performed by using Genome Walker Kit (Clontech), because it seemed that pAOX514 might not have sufficient length to contain promoter for *AOX* gene. At first, the PCR primers whose sequences were shown in TABLE 5 were synthesized.

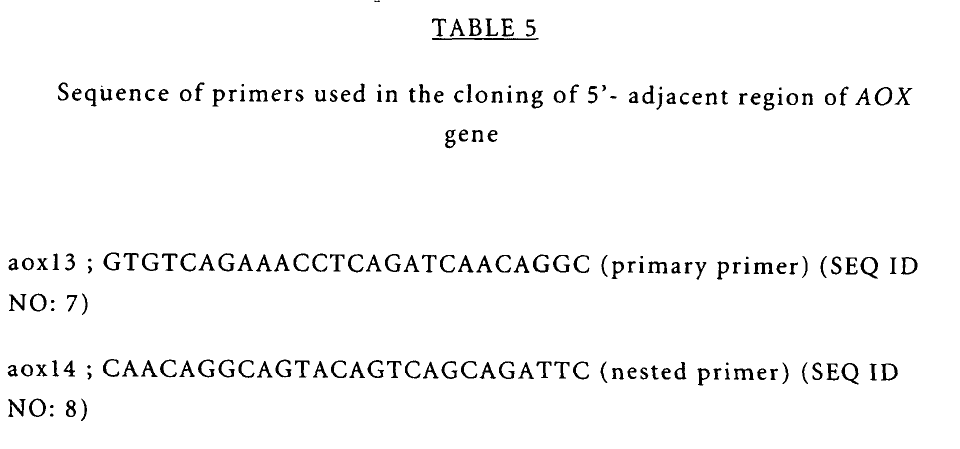

Protocols for library construction and PCR condition were the same as those specified by the manufacturer. The genomic DNA preparation obtained in Example 5 was used as a PCR template. The PCR fragments that had *Sca*I site at the 5'- end (1.2 kb), and that had *Dra*I site at the 5'-end (3.0 kb) were recovered and cloned into pCR2.1-TOPO by using *E*. *coli* TOP10 as a host strain. As a result of sequencing of each 2 independent clones from both constructs, it was confirmed that the 5'-adjacent region of *AOX* gene was cloned. The clone obtained by the ScaI construct in the above experiment was designated as pAOXSc702 and used for further study. Based on the sequence of insert fragment in pAOXSc702, 4 PCR primers whose sequence are listed in TABLE 6 were synthesized.

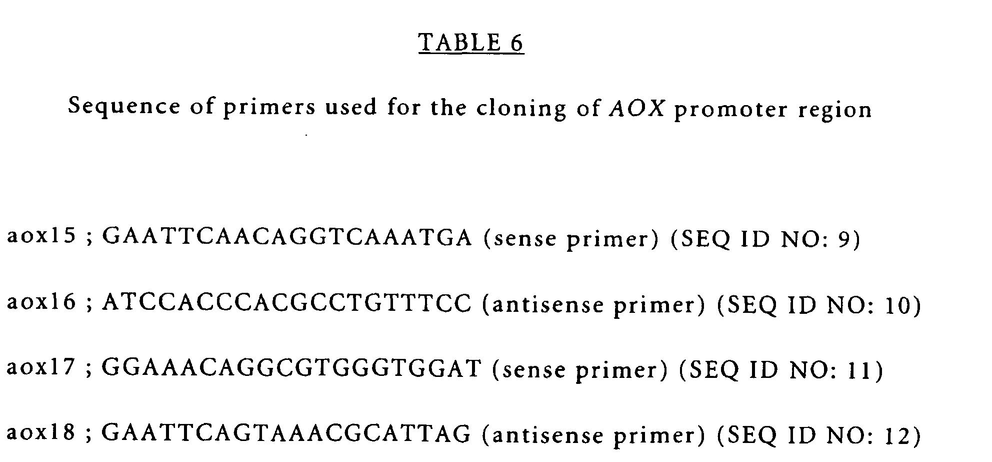

The PCR condition was the same as shown in Example 4 except that HF polymerase (Clontech) was used as DNA polymerase. In the combination of aox15 and aox16, the fragment which has 0.7 kb in its length was amplified. In the combination of aox17 and aox18, the fragment which has 0.5 kb in its length was amplified. These fragments were cloned into pCR2.1-TOPO and transformed *E*. *coli* TOP10. Plasmids were prepared from 6 independent white colonies, respectively and subjected to the sequencing. As a result, expected clones whose sequence of the insert fragment were identical to each other were obtained. In the combination of aox15 and aox16, obtained clone was named as pAOX714 #1516. In the combination of aox17 and aox18, obtained clone was named as pAOX714 #1718. As a result of sequencing, sequence of pAOX714 #1516 and pAOX714 #1718 which contained promoter region for *AOX* gene from *P*. *rhodozyma* were determined. Determined sequence containing AOX promoter was 1406 base pairs in its length.

Combining the sequence which was obtained in Example 8 and 9, it was determined that the nucleotide sequence (3.7 kb) contained *AOX* gene and its promoter and terminator (SEQ ID NO: 2).

### Example 10 Construction of antisense plasmid for AOX gene

An antisense gene fragment which covered the entire structure gene for *AOX* gene was amplified by PCR method and then cloned into integration vector in which antisense AOX gene was transcribed by *AST* promoter in *P. rhodozyma*.

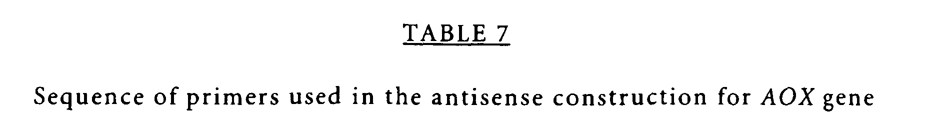

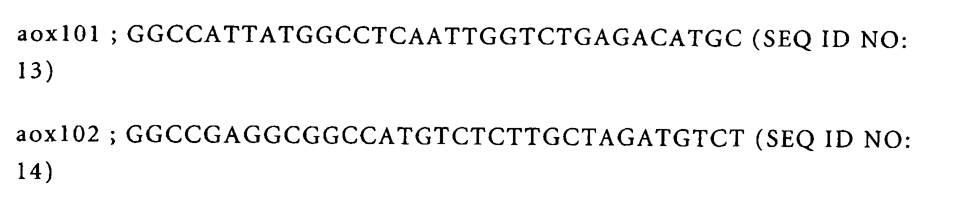

Both primers, aox101 and 102 had asymmetrical recognition sequence for restriction enzyme, SfiI (GGCCNNNNNGGCC) but their asymmetrical hang-over sequence was designed to be different. This might enable a directional cloning into expression vector which has the same asymmetrical sequence at their ligation sequence.

PCR was performed by using HF polymerase (Clontech) as Taq polymerase and the cDNAs prepared in Example 3 as a template under the condition as follows; 30 cycles of 94°C for 15 seconds, 55°C for 30 seconds and 72°C for 45 seconds. The amplified PCR fragment was purified and cloned into pCR2.1-TOPO vector. As a result of sequencing, it was revealed that one clone had the correct fragment and this clone was named as pAOX1007 #0102. The sequence of antisense fragment for *AOX* gene is listed in SEQ ID NO: 15.

For the promoter and terminator fragment which drove the transcription of the antisense *AOX* gene, *AST* promoter and terminator were cloned from the chromosome prepared in Example 5.

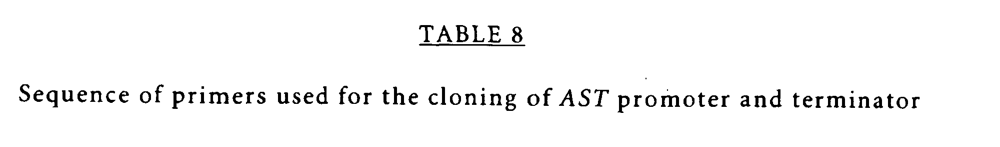

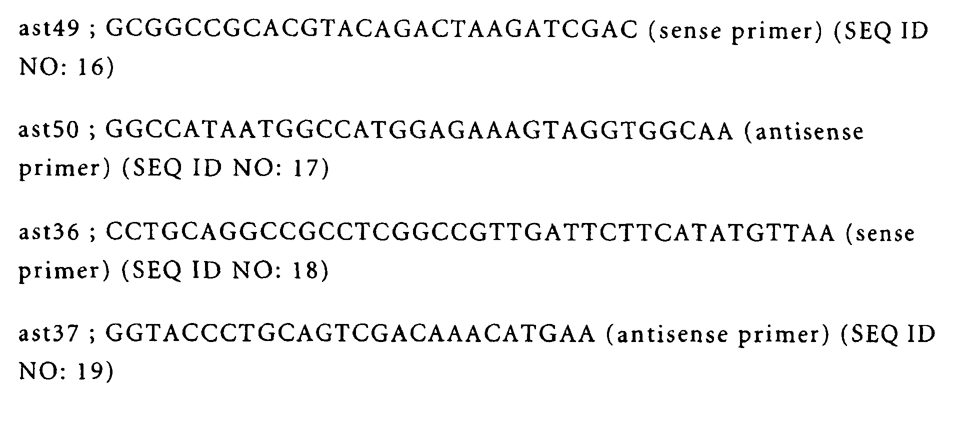

The PCR condition was as follows; 25 cycles of 94 for 15 seconds, 55°C for 30 seconds and 72°C for 90 seconds. In the combination of ast49 and ast50, the fragment which has 1.25 kb in its length was amplified. In the combination of ast36 and ast37, the fragment which has 0.3 kb in its length was amplified. These fragments were cloned into pCR2.1-TOPO and transformed *E*. *coli* TOP10. Plasmids were prepared from 6 independent white colonies, respectively and subjected to the sequencing. As a result, the clones that had correct sequence of *AST* promoter and terminator (EP 1 035 206 A1) were selected for further study (pUAST407 for *AST* promoter and pAST526 #3637 for *AST* terminator).

Next, AST terminator sequence was fused to G418 resistant cassette by ligating *Not*I- plus *Kpn*I*-* digested pAST526 #3637 and *Kpn*I*-* plus *Sac*I-digested pG418Sa330 (EP 1 035 206 A1) to *Not*I*-* and *Sac*I*-* digested pBluescriptII SK- (Stratagene). The ligation mixture was transformed into competent KB822 cells. As a result of restriction analysis, one clone (pUAST418) which had the correct structure was selected for further study.

Then, 3.1 kb of SacI fragment containing ribosomal DNA (rDNA) locus (Wery *et al*., Gene, 184, 89-97, 1997) was inserted in the downstream of G418 cassette of pUAST418. rDNA fragment exists in multicopies on the chromosome of Eukaryote. The integration event via the rDNA fragment results in multicopied integration onto the chromosome of the host used and this enables the overexpression of foreign genes which are harbored in expression vector. For this purpose, SacI fragment from pGBPh9 containing rDNA gene was ligated to SacI-digested and bacterial alkaline phosphatase-treated pUAST418. The ligation mixture was transformed into competent KB822 cells. As a result of restriction analysis, two clones, in which rDNA fragment was inserted in different orientation each other were selected for further study (pURDNA421 and pURDNAR421).

Subsequently, *AST* promoter was inserted in the upstream of *AST* terminator to construct of expression vector which functions in *P. rhodozyma*. 1.0kb of *Not*I*-* and *Bgl*II*-* fragment of pUAST407 and 0.25kb fragment of *Bgl*II and *Pst*I fragment of pUAST407 were ligated to *Not*I*-* and Sse8387I- digested pURDNA421 or pURDNAR421. The competent KB822 cells were transformed by the ligation mixture and each 6 resultant colonies were subjected to restriction analysis. The clones which had the correct insertion of *AST* promoter were selected for further study (pF718 and pR718 which had the opposite orientation of rDNA fragment) to each other.

Finally, antisense *AOX* construct was completed by inserting the 1.2kb of *Sfi*I fragment of pAOX1007#0102 into the SfiI-digested pF718 or pR718. The resultant plasmids were named as pFAOX828 and pRAOX828.

### Example 11 Transformation of P. rhodozyma with AOX-antisense vector

The *AOX*-antisense vectors, pFAOX828 and pRAOX828 were transformed into *P*. *rhodozyma* wild type strain, ATCC96594. Biolistic transformation was performed according to the method described in Methods in Molecular Biology (Johnson *et al*., 53, 147-153, 1996). *P. rhodozyma* strain, ATCC96594 was cultured in YPD medium to the stationary phase. After centrifugation of the broth, cells were concentrated by 10-fold with sterilized water and 200 microliter of the cell suspension was spread on YPD medium containing 100 microgram/ml of geneticin, and 0.75M of D-mannitol and D-sorbitol. Five microgram of plasmids was coated on 1.5 mg of 0.9 micrometer gold particle, and used as donor DNAs for the Biolistic transformation. One geneticin resistant colony which was transformed with pFAOX828 and showed enhanced pigmentation was selected for further characterization in view of its productivity of astaxanthin and its decreased activity of alternative oxidase which was encoded by *AOX* gene.

### Example 12 Characterization of the pFAOX828 integrant derived from P. rhodozyma, ATCC96594

The *P. rhodozyma* transformant, ATCC96594 :: pFAOX828, together with its parent strain ATCC96594 was cultured in 50 ml of YPD medium in 500 ml Erlenmeyer flask at 20 °C for 3 days by using their seed culture which grew in 10 ml of YPD medium in test tubes (21 mm in diameter) at 20 °C for 3 days. At different time points, e.g. at 24, 43 and 65 hours after the inoculation, appropriate volume of culture broth was withdrawn and used for analysis of their growth, productivity of astaxanthin and their oxygen uptake activity under the presence or absence of KCN. The 24, 43 and 65 hour-time point corresponded to their growth phase of late log-phase, mid-stationary and late stationary, respectively.

For analysis of growth, optical density at 660 nm was measured by using UV-1200 photometer (Shimadzu Corp., Kyoto, Japan) in addition to the determination of their dried cell mass by drying up the cells derived from 1 ml of broth after microcentrifugation at 100 °C for one day.

For analysis of content of astaxanthin and total carotenoids, cells were harvested from 1.0 ml of broth after microcentrifugation and used for the extraction of the carotenoids from cells of *P*. *rhodozyma* by disruption with glass beads. After extraction, disrupted cells were then removed by centrifugation and the resultant was analyzed for carotenoid content with HPLC. The HPLC condition used was as follows;
HPLC column; Chrompack Lichrosorb si-60 (4.6 mm, 250 mm)
Temperature; room temperature
Eluent; acetone / hexane (18/82) add 1 ml/L of water to eluent
Injection volume; 10 microliter
Flow rate; 2.0 ml/minute
Detection; UV at 450 nm

A reference sample of astaxanthin was obtained from F. Hoffmann-La Roche AG (Basel, Switzerland).

For determination of respiration activity by measuring oxygen uptake activity in the presence or absence of KCN, the DO meter model, B-505 and the DO probe, GU-BM manufactured by Iijima Electronics Corporation (Aichi, Japan) were used. Harvested cells were resuspended with 0.5 M KPB (pH 7.4). Then, 200 microliter of this cell suspension was diluted with 2.3 ml of water in the chamber of DO analysis. The measurement was initiated by the addition of 0.2 ml of 1 M glucose in the presence or absence of 0.48 mM KCN.

Results are summarized in TABLE 9.

**TABLE 9**

| strain | ATCC96594 :: pFAOX828 | | | ATCC96594 | | |
|---|---|---|---|---|---|---|
| times (hours) | 24 | 43 | 65 | 24 | 43 | 65 |
| OD at 660nm | 22.75 | 28.26 | 27.91 | 28.75 | 31.707 | 31.10 |
| dried cells (mg/ml) | 10.8 | 12.7 | 12.3 | 11.6 | 12.1 | 11.6 |
| astaxanthin (mg/g-dried cells) | 0.090 | 0.223 | 0.240 | 0.107 | 0.194 | 0.211 |
| carotenoids (mg/g-dried cells) | 0.218 | 0.336 | 0.354 | 0.213 | 0.269 | 0.288 |
| respiration | | | | | | |
| - KCN sensitive | 11.29 | 5.84 | 4.02 | 16.03 | 6.13 | 4.96 |
| - KCN resistant | 0.29 | 0 | 0 | 1.48 | 0.78 | 0.15 |

### (Activity of respiration is expressed by nmol O₂-uptake / min x mg-dried cells.)

As shown in TABLE 9, antisense *AOX* transformant ATCC96594 :: pFAOX828 showed similar cell yield to parent strain, ATCC96594 at 43 hours of culture although it showed slower growth at 24 hours. Astaxanthin and carotenoid content of the transformant increased by 15 %. In view of its respiration activity, the transformant had the similar activity of KCN-sensitive respiration to its host strain (95 %) but KCN-resistant respiration mediated by alternative oxidase was decreased to 20 % levels of its host strain at 24 hours culture and perfectly impaired at 43 hours of culture.

From this result, it was indicated that the decrease of alternative oxidase activity which mediated KCN-resistant respiration might lead to the overproduction of astaxanthin and carotenoids in *P. rhodozyma*.

## Claims

1. A process for producing carotenoids which comprises treating a parent organism capable to produce carotenoids to induce a reduction of an alternate oxidase activity and selecting an organism which production of carotenoids is enhanced, and cultivating the selected organism.

2. A process according to claim 1, wherein the selected organism is a mutant strain of which productivity of carotenoids is enhanced with the aid of alteration of the resistance against an alternative oxidase inhibitor.

3. A process according to claim 1 or 2, wherein the selected organism is a mutant resistant to an alternative oxidase inhibitor.

4. A process according to claim 2 or 3, wherein the said organism belongs to the kingdom of Protista or Fungi*,* more preferably to the genus *Synechococcus*, *Synechocystis*, Haematococcus, Dunaliella, *Phaffia*, Xanthophyllomyces, Neurospora, Rhodotorula, Blakeslea, or Phycomyces.

5. A process according to any one of claims 2 to 4, wherein the said organism is a strain of *Phaffia rhodozyma*.

6. A process according to any one of claims 2 to 5, wherein the said organism is a strain selected from the group consisting of DSM 13429, 13430 and 13431.

7. A process according to any one of claims 2 to 6, wherein the alternative oxidase inhibitor is selected from the group consisting of n-propyl gallate and salicylhydroxamic acid.

8. A method for establishing a mutant strain capable of producing carotenoids in an enhanced level relative to a parent organism, which comprises cultivating a parent organism capable to produce carotenoids under the condition for reducing an alternative oxidase activity and selecting an organism capable of producing carotenoids in a level higher than the said parent organism.

9. A method according to claim 8, wherein the condition for reducing an alternative oxidase activity comprises the presence of an alternative oxidase inhibitor.

10. A method according to claim 9, wherein the alternative oxidase inhibitor is selected from the group consisting of n-propyl gallate and salicylhydroxamic acid.

11. A method according to any one of claims 8 to 10, wherein the said organism belongs to the kingdom of Protista or Fungi*,* more preferably to the genus *Synechococcus*, Synechocystis, Haematococcus, Dunaliella, *Phaffia*, Xanthophyllomyces, Neurospora, Rhodotorula, Blakeslea, or Phycomyces.

12. A method according to any one of claims 8 to 11, wherein the said organism is a strain of *Phaffia rhodozyma*.

13. A method according to any one of claims 8 to 12, wherein the selected organism is a strain from the group consisting of DSM 13429, 13430 and 13431.

14. A process according to claim 1, wherein the selected organism is a recombinant organism and wherein gene expression of the alternative oxidase is altered to reduce efficiency compared to a host organism.

15. A process according to claim 14, wherein the gene expression of the alternative oxidase is altered with the aid of the technique selected from antisense technology, site-directed mutagenesis, chemical mutagenesis, etc.

16. A process according to claim 14 or 15, wherein the said organism belongs to the kingdom of Protista or Fungi*,* more preferably to the genus *Synechococcus*, Synechocystis, Haematococcus, Dunaliella, Phaffia, Xanthophyllomyces, Neurospara, Rhodotorula, Blakeslea, or Phycomyces.

17. A process according to any one of claims 14 to 16, wherein the said organism is a strain of *Phaffia rhodozyma*.

18. A recombinant organism capable of producing carotenoids in an enhanced level relative to its parent organism which is **characterized in that** gene expression of the alternative oxidase is altered to reduce efficency compared to the parent organism, wherein the said organism belongs to the genus Synechococcus, Synechocystis, Haematococcus, Dunaliella, Phaffia, Xanthophyllomyces, Neurospara, Rhodotorula, Blakeslea, or Phycomyces.

19. A recombinant organism according to claim 18, wherein the gene expression of the alternative oxidase is altered with the aid of the technique selected from antisense technology, site-directed mutagenesis, chemical mutagenesis, etc.

20. A recombinant organism according to any one of claims 18 to 19, wherein the said organism is a strain of *Phaffia rhodozyma*.

21. A recombinant DNA sequence coding for an alternative oxidase derived from an organism producible of carotenoids, wherein the said organism is a strain of Phaffia rhodozyma and the said sequence is SEQ ID NO: 2 or has identity with SEQ ID NO: 2 higher than 55%.

22. A recombinant DNA sequence according to claim 21, whose deduced amino acid sequence is SEQ ID NO: 1 or has identity with SEQ ID NO: 1 higher than 51.5%.

## Patentansprüche

1. Verfahren zur Herstellung von Carotinoiden, umfassend die Behandlung eines Elternorganismus, der Carotinoide produzieren kann, um eine Reduktion einer alternativen Oxidaseaktivität zu induzieren, und Auswählen eines Organismus, dessen Produktion von Carotinoiden erhöht ist, und Kultivieren des ausgewählten Organismus.

2. Verfahren nach Anspruch 1, wobei der ausgewählte Organismus ein Mutantenstamm ist, dessen Carotinoidproduktivität mit Hilfe der Alteration der Resistenz gegenüber einem alternative Oxidase-Inhibitor gesteigert wird.

3. Verfahren nach Anspruch 1 oder 2, wobei der ausgewählte Organismus eine Mutante ist, die gegen einen alternative Oxidase-Inhibitor resistent ist.

4. Verfahren nach Anspruch 2 oder 3, wobei der Organismus zu dem Reich der Protista oder Fungi, stärker bevorzugt zu der Gattung Synechococcus, Synechocystis, Haematococcus, Dunaliella, Phaffia, Xanthophyllomyces, Neurospora, Rhodotorula, Blakeslea oder Phycomyces gehört.

5. Verfahren nach einem der Ansprüche 2 bis 4, wobei der Organismus ein Stamm von Phaffia rhodozyma ist.

6. Verfahren nach einem der Ansprüche 2 bis 5, wobei der Organismus ein Stamm ist, ausgewählt aus der Gruppe, bestehend aus DSM, 13429, 13430 und 13431.

7. Verfahren nach einem der Ansprüche 2 bis 6, wobei der alternative Oxidase-Inhibitor aus der Gruppe, bestehend aus n-Propylgallat und Salicylhydroxamsäure, ausgewählt ist.

8. Verfahren zur Herstellung eines Mutantenstammes, der Carotinoide in einem erhöhtem Niveau in bezug auf den Elternorganismus produzieren kann, umfassend das Kultivieren eines Elternorganismus, der Carotinoide produzieren kann, unter der Bedingung zur Reduktion einer alternativen Oxidaseaktivität und Auswählen eines Organismus, der Carotinoide in einem höheren Niveau als der Elternorganismus produzieren kann.

9. Verfahren nach Anspruch 8, wobei die Bedingung zur Reduktion einer alternativen Oxidaseaktivität die Gegenwart eines alternative Oxidase-Inhibitors umfaßt.

10. Verfahren nach Anspruch 9, wobei der alternative Oxidase-Inhibitor aus der Gruppe, bestehend aus n-Propylgallat und Salicylhydroxamsäure, ausgewählt ist.

11. Verfahren nach einem der Ansprüche 8 bis 10, wobei der Organismus zu dem Reich der Protista oder Fungi, stärker bevorzugt zu der Gattung Synechococcus, Synechocystis, Haematococcus, Dunaliella, Phaffia, Xanthophyllomyces, Neurospora, Rhodotorula, Blakeslea oder Phycomyces gehört.

12. Verfahren nach einem der Ansprüche 8 bis 11, wobei der Organismus ein Stamm von Phaffia rhodozyma ist.

13. Verfahren nach einem der Ansprüche 8 bis 12, wobei der ausgewählte Organismus ein Stamm aus der Gruppe, bestehend aus DSM, 13429, 13430 und 13431, ist.

14. Verfahren nach Anspruch 1, wobei der ausgewählte Organismus ein rekombinanter Organismus ist, und wobei die Genexpression der alternativen Oxidase verändert wird, um die Wirksamkeit im Vergleich zu dem Wirtsorganismus zu verringern.

15. Verfahren nach Anspruch 14, wobei die Genexpression der alternativen Oxidase mit Hilfe der Technik, ausgewählt aus Antisense-Technologie, ortsgerichteter Mutagenese, chemischer Mutagenese usw., verändert wird.

16. Verfahren nach Anspruch 14 oder 15, wobei der Organismus zu dem Reich der Protista oder Fungi, stärker bevorzugt zu der Gattung Synechococcus, Synechocystis, Haematococcus, Dunaliella, Phaffia, Xanthophyllomyces, Neurospora, Rhodotorula, Blakeslea oder Phycomyces gehört.

17. Verfahren nach einem der Ansprüche 14 bis 16, wobei der Organismus ein Stamm von Phaffia rhodozyma ist.

18. Rekombinanter Organismus, der Carotinoide in einem erhöhtem Niveau in bezug auf seinen Elternorganismus produzieren kann, der **dadurch gekennzeichnet ist, daß** die Genexpression der alternativen Oxidase verändert ist, um die Wirksamkeit im Vergleich zu dem Elternorganismus zu verringern, wobei der Organismus zu der Gattung Synechococcus, Synechocystis, Haematococcus, Dunaliella, Phaffia, Xanthophyllomyces, Neurospora, Rhodotorula, Blakeslea oder Phycomyces gehört.

19. Rekombinanter Organismus nach Anspruch 18, wobei die Genexpression der alternativen Oxidase mit Hilfe der Technik, ausgewählt aus Antisense-Technologie, ortsgerichteter Mutagenese, chemischer Mutagenese usw., verändert wird.

20. Rekombinanter Organismus nach einem der Ansprüche 18 bis 19, wobei der Organismus ein Stamm von Phaffia rhodozyma ist.

21. Rekombinante DNA-Sequenz, die für eine alternative Oxidase kodiert, die aus einem Organismus stammt, der Carotinoide produzieren kann, wobei der Organismus ein Stamm von Phaffia rhodozyma ist und die Sequenz SEQ ID Nr: 2 ist, oder eine Identität mit SEQ ID Nr: 2 von höher als 55 % aufweist.

22. Rekombinante DNA-Sequenz nach Anspruch 21, deren abgeleitete Aminosäuresequenz SEQ ID Nr:1 ist oder eine Identität mit SEQ ID Nr. 1 von höher als 51,5 % aufweist

## Revendications

1. Procédé de production de caroténoïdes qui comprend le traitement d'un organisme parent capable de produire des caroténoïdes pour induire une réduction d'une activité d'oxydase alternative et la sélection d'un organisme dont la production de caroténoïdes est améliorée, et la culture de l'organisme sélectionné.

2. Procédé selon la revendication 1, dans lequel l'organisme sélectionné est une souche mutante dont la productivité en caroténoïdes est améliorée au moyen de l'altération de la résistance à un inhibiteur de l'oxydase alternative.

3. Procédé selon la revendication 1 ou 2, dans lequel l'organisme sélectionné est un mutant résistant à un inhibiteur de l'oxydase alternative.

4. Procédé selon la revendication 2 ou 3, dans lequel ledit organisme appartient au règne des protistes ou des champignons, de manière plus particulièrement préférée au genre *Synechococcus, Synechocystis,* Haematococcus, *Dunaliella, Phaffia, Xanthophyllomyces, Neurospora, Rhodotorula, Blakeslea* ou *Phycomyces.*

5. procédé selon l'une quelconque des revendications 2 à 4, dans lequel ledit organisme est une souche de *Phaffia rhodozyma.*

6. Procédé selon l'une quelconque des revendications 2 à 5, dans lequel ledit organisme est une souche choisie dans le groupe constitué par DSM 13429, 13430 et 13431.

7. Procédé selon l'une quelconque des revendications 2 à 6, dans lequel l'inhibiteur de l'oxydase alternative est choisi dans le groupe constitué par le n-propylgallate et l'acide salicylhydroxamique.

8. Procédé d'établissement d'une souche mutante capable de produire des caroténoides en quantité améliorée par rapport à un organisme parent, comprenant la culture d'un organisme parent capable de produire des caroténoïdes dans des conditions de réduction d'une activité d'oxydase alternative, et la sélection d'un organisme capable de produire des caroténoïdes en quantité supérieure par rapport audit organisme parent.

9. Procédé selon la revendication 8, dans lequel les conditions de réduction d'une activité d'oxydase alternative comprennent la présence d'un inhibiteur de l'oxydase alternative.

10. Procédé selon la revendication 9, dans lequel l'inhibiteur de l'oxydase alternative est choisi dans le groupe constitué par le n-propylgallate et l'acide salicylhydroxamique.

11. Procédé selon l'une quelconque des revendications 8 à 10, dans lequel ledit organisme appartient au règne des protistes ou des champignons, de manière plus particulièrement préférée au genre *Synechococcus, Synechocystis, Haematococcus, Dunaliella, Phaffia, Xanthophyllomyces, Neurospora, Rhodotorula, Blakeslea* ou *Phycomyces.*

12. Procédé selon l'une quelconque des revendications 8 à 11, dans lequel ledit organisme est une souche de *Phaffia rhodozyma.*

13. Procédé selon l'une quelconque des revendications 8 à 12, dans lequel l'organisme sélectionné est une souche du groupe constitué par DSM 13429, 13430 et 13431.

14. , Procédé selon la revendication 1, dans lequel l'organisme sélectionné est un organisme recombinant et dans lequel l'expression génique de l'oxydase alternative est altérée pour que sont efficacité soit réduite par rapport à celle d'un organisme hôte.

15. Procédé selon la revendication 14, dans lequel l'expression génique de l'oxydase alternative est altérée au moyen d'une Procédé choisie parmi la Procédé anti-sens, la mutagénèse dirigée, la mutagénèse chimique, etc.

16. Procédé selon la revendication 14 ou 15, dans lequel ledit organisme appartient au règne des protistes ou des champignons, de manière plus particulièrement préférée au genre *Synechococcus*, *Synechocystis, Haematococcus,* Dunaliella, *Phaffia, Xanthophyllomyces,* Neurospora, Rhodotorula, *Blakeslea* ou Phycomyces.

17. Procédé selon l'une quelconque des revendications 14 à 16, dans lequel ledit organisme est une souche de *Phaffia rhodozyma.*

18. Organisme recombinant capable de produire des caroténoïdes en quantité améliorée par rapport à son organisme parent, qui est **caractérisé en ce que** l'expression génique de l'oxydase alternative est altérée pour que son efficacité soit réduite par rapport à celle de l'organisme parent, dans lequel ledit organisme appartient au genre Synecnococcus, *Synechocystis,* Haematococcus, Dunaliella, Phaffia, *Xanthophyllomyces,* Neurospora, *Rhodotorula, Blakeslea* ou *Phycomyces.*

19. Organisme recombinant selon la revendication 18, dans lequel l'expression génique de l'oxydase alternative est altérée au moyen d'une Procédé choisie parmi la Procédé anti-sens, la mutagénèse dirigée, la mutagénèse chimique, etc.

20. Organisme recombinant selon l'une quelconque des revendications 18 ou 19, dans lequel ledit organisme est une souche de *Phaffia rhodozyma.*

21. Séquence d'ADN recombinant codant une oxydase alternative dérivée d'un organisme producteur de caroténoïdes, dans laquelle ledit organisme est une souche de *Phaffia rhodozyma* et ladite séquence est SEQ ID N° 2 ou présente une identité supérieure à 55% avec SEQ ID N° 2.

22. , Séquence d'ADN recombinant selon la revendication 21, dont la séquence d'acides aminés déduite est SEQ ID N° 1 ou présente une identité supérieure à 51,5% avec SEQ ID N° 1.


### SEQUENCE LISTING

<110> F. HOFFMANN-LA ROCHE AG
<120> Improvement of biological production of carotenoids and biological materials therefor
<130> Case 20685

<140> -
<141> -

<150> 00111148.3
<151> 2000-05-24

<160> 12
<170> PatentIn Ver. 2.0

<210> 1
<211> 401
<212> PRT
<213> Phaffia rhodozyma

<400> 1
   Met Ser Leu Ala Arg Cys Leu Val Gln Ala Ser Thr Arg Ser Leu
   1               5                   10                  15
   Ser Arg Thr Val Arg Pro Ser Tyr Leu Thr Pro Leu Thr Val His
                   20                  25                  30
   Phe Phe Ser Ser Thr Ile Ser Arg Ser Cys Ser Arg Ser Tyr Ser
                   35                  40                  45
   Thr Ser Asn Thr Arg Leu Ser Thr Ser Asn Gly Gln Gln Ser Thr
                   50                  55                  60
   His His Leu Ala Asp Asn Val Pro Leu Thr Thr Asp Lys Gln Arg
                   65                  70                  75
   His Leu Gln Gly Val Ile Gly Gly Glu Gly Met His Gln His Asp
                   80                  85                  90
   Ala Thr Thr Val Ala His Thr Asp Pro Leu Ala Ser Val Ile Gln
                   95                  100                 105
   Asp Leu Thr Val Pro Thr Asn Gly Ser Trp Val Met His Asn Pro
                   110                 115                 120
   Val Tyr Thr Arg Thr Glu Leu Asp Ala Val Gln Val Val His Arg
                   125                 130                 135
   Pro Pro Thr Asn Thr Ser Asp Gln Val Ser Thr Lys Leu Val Lys
                   140                 145                 150
   Met Leu Arg Trp Gly Phe Asp Leu Val Ser Asn Tyr Lys His Val
                   155                 160                 165
   Pro Phe Pro Ala Asn His Lys Glu Leu Ser Val Thr Gln Leu Arg
                   170                 175                 180
   Gln Met Gly Cys Leu Leu Ser Pro Asp Gln Trp Met Thr Arg Phe
                   185                 190                 195
   Ile Phe Leu Glu Thr Thr Ala Ala Ile Pro Gly Met Val Gly Gly
                   200                 205                 210
   Leu Leu Arg His Leu Gln Ser Leu Arg Leu Met Arg Arg Asp Gly
                   215                 220                 225
   Gly Trp Ile His Thr Leu Leu Ala Glu Ala Glu Asn Glu Arg Leu
                   230                 235                 240
   His Leu Leu Thr Phe Met Ser Met Ala Asn Pro Pro Leu Trp Phe
                   245                 250                 255
   Arg Ala Leu Ile Leu Gly Ala Gln Gly Val Phe Tyr Asn Leu Phe
                   260                 265                 270
   Phe Ile Thr Tyr Leu Ile Ser Pro Pro Val Ala His Arg Phe Val
                   275                 280                 285
   Ala Cys Leu Glu Glu Glu Ala Val Val Thr Tyr Thr Arg Ile Ile
                   290                 295                 300
   Ser Asp Ile Glu Asn Gly Tyr Val Pro Glu Trp Glu Lys Leu Pro
                   305                 310                 315
   Ala Pro Glu Ile Ala Ile Ser Tyr Trp Arg Leu Pro Pro Asp Ala
                   320                 325                 330
   Thr Phe Leu Asp Thr Leu Arg Ala Ile Arg Ala Asp Glu Ala Thr
                   335                 340                 345
   His Arg Phe Val Asn His Thr Phe Ala Ser Leu Asp Ser Lys Lys
                   350                 355                 360
   Asp Phe Asn Pro Phe Ala Ile Ala Glu Pro Asp Ala Thr Thr Lys
                   365                 370                 375
   Gly Ser Val Tyr Gly Phe Thr Arg Asp Glu Ala Ala Ala Phe Ala
                   380                 385                 390
   Gln Lys Thr Arg Glu Arg Met Ser Gln Thr Asn
                   395                 400

<210> 2
<211> 3724
<212> DNA
<213> Phaffia rhodozyma

<220>
<221> exon
<222> (1407)..(1674)

<220>
<221> intron
<222> (1675)..(1769)

<220>
<221> exon
<222> (1770)..(1873)

<220>
<221> intron
<222> (1874)..(1948)

<220>
<221> exon
<222> (1949)..(2155)

<220>
<221> intron
<222> (2156)..(2264)

<220>
<221> exon
<222> (2265)..(2295)

<220>
<221> intron
<222> (2296)..(2372)

<220>
<221> exon
<222> (2373)..(2423)

<220>
<221> intron
<222> (2424)..(2515)

<220>
<221> exon
<222> (2516)..(2645)

<220>
<221> intron
<222> (2646)..(2735)

<220>
<221> exon
<222> (2736)..(2831)

<220>
<221> intron
<222> (2832)..(2914)

<220>
<221> exon
<222> (2915)..(2998)

<220>
<221> intron
<222> (2999)..(3085)

<220>
<221> exon
<222> (3086)..(3206)

<220>
<221> intron
<222> (3207)..(3320)

<220>
<221> exon
<222> (3321)..(3434)

<220>
<221> 5'UTR
<222> (1295)..(1296)
<223> (EXPERIMENTAL)

<220>
<221> polyA_site
<222> (3682)..(3683)
<223> (EXPERIMENTAL)

<400> 2

<210> 3
<211> 26
<212> DNA
<213> Artificial Sequence
<220>
<223> Description of Artificial Sequence:degenerate PCR primer(sense primer)

<400> 3
   aaygarmgna tgcayytnyt nacntt 26

<210> 4
<211> 26
<212> DNA
<213> Artificial Sequence

<220>
<223> Description of Artificial Sequence:degenerate PCR primer (antisense primer)

<400> 4
   gcytcytcyt cnarrtancc nacraa 26

<210> 5
<211> 20
<212> DNA
<213> Artificial Sequence

<220>
<223> Description of Artificial Sequence:sequencing primer (5' nucleotide was Cy5-labeled)

<400> 5
   agctacaaca tacgaaaggg 20

<210> 6
<211> 20
<212> DNA
<213> Artificial Sequence

<220>
<223> Description of Artificial Sequence:sequencing primer (5' nucleotide was Cy5-labeled)

<400> 6
   ggggaactga gagctctaaa 20

<210> 7
<211> 26
<212> DNA
<213> Artificial Sequence

<220>
<223> Description of Artificial Sequence:genome walking PCR primer (primary primer)

<400> 7
   gtgtcagaaa cctcagatca acaggc 26
<210> 8

<211> 26
<212> DNA
<213> Artificial Sequence

<220>
<223> Description of Artificial Sequence:genome walking PCR primer (nested primer)

<400> 8
   caacaggcag tacagtcagc agattc 26

<210> 9
<211> 20
<212> DNA
<213> Artificial Sequence

<220>
<223> Description of Artificial Sequence:PCR primer for cloning of promoter region (sense primer)

<400> 9
   gaattcaaca ggtcaaatga 20

<210> 10
<211> 20
<212> DNA
<213> Artificial Sequence

<220>
<223> Description of Artificial Sequence:PCR primer for cloning of promoter region (antisense primer)

<400> 10
   atccacccac gcctgtttcc 20

<210> 11
<211> 20
<212> DNA
<213> Artificial Sequence

<220>
<223> Description of Artificial Sequence:PCR primer for cloning of promoter region (sense primer)

<400> 11
   ggaaacaggc gtgggtggat 20

<210> 12
<211> 20
<212> DNA
<213> Artificial Sequence

<220>
<223> Description of Artificial Sequence:PCR primer for cloning of promoter region (antisense primer)

<400> 12
   gaattcagta aacgcattag 20

<210> 13
<211> 33
<212> DNA
<213> Artificial Sequence

<220>
<223> Description of Artificial Sequence:PCR primer for construction of antisense AOX gene (sense primer)

<400> 13
   ggccattatg gcctcaattg gtctgagaca tgc 33

<210> 14
<211> 33
<212> DNA
<213> Artificial Sequence

<220>
<223> Description of Artificial Sequence:PCR primer for construction of antisense AOX gene (antisense primer)

<400> 14
   ggccgaggcg gccatgtctc ttgctagatg tct 33

<210> 15
<211> 1232
<212> DNA
<213> Phaffia rhodozyma

<400> 15
   ggccattatg gcctcaattg gtctgagaca tgcgttctct cgtcttctga      50
   gcgaaggcgg cggcctcgtc ccgtgtgaaa ccatataccg agcctttagt     100
   agtggcgtct ggctcggcta tcgcaaatgg attgaagtct ttcttagagt     150
   ccaggctggc aaatgtgtga ttcacgaatc gatgagtggc ctcatctgct     200
   cggatggctc gcagtgtgtc caaaaaggta gcatcgggag gaagtcgcca     250
   gtaagatata gcaatctcgg gagcgggaag cttttcccat tcaggtacat     300
   agccgttctc gatatcactg ataattcttg tgtaagtaac gacagcttct     350
   tcctccaggc aggcaacgaa tcgatgagcc accggcgggg aaattaaata     400
   agttatgaag aacaggttat aaaaaacccc ttgagctccc agtatcaaag     450
   ctcggaacca gagaggtgga ttagccatgc tcatgaacgt cagaaggtgg     500
   agacgttcgt tttcagcttc agcaagaagc gtgtgaatcc aaccaccatc     550
   ccgtcgcatg agtcggagag actgaagatg gcgcaagaga ccgccaacca     600
   ttccaggaat agcagctgtt gtttctagaa agatgaacct cgtcatccat     650
   tgatcaggcg agagaagaca gcccatttgg cgcaattgag tgacgctgag     700
   ttctttgtgg tttgcgggaa agggaacatg tttgtagttg ctgacaaggt     750
   cgaatcccca tcggagcatc ttgacaagct tggtggagac ttggtcggac     800
   gtgttggtgg ggggacgatg aacgacctga acagcatcta actcagttcg     850
   agtatagacg ggattatgca tcacccaaga tccgttagtg ggaacagtca     900
   aatcttgtat gacggaagct aagggatccg tatgagctac cgtcgttgca     950
   tcatgctgat gcatgccctc accgccgatg acgccttgaa ggtgcctttg    1000
   tttgtcggtg gtgagaggaa cattgtccgc aagatgatgc gttgattgtt    1050
   gaccattaga tgttgaaagg cgggtgttcg acgttgaata tgacctggag    1100
   cagctccttg agattgttga ggagaagaag tgaactgtta gaggtgtgag    1150
   ataggatggc cgaacggtac gcgaaagtga ccgagttgat gcctggacaa    1200
   gacatctagc aagagacatg gccgcctcgg cc                       1232

<210> 16
<211> 28
<212> DNA
<213> Artificial Sequence

<220>
<223> Description of Artificial Sequence: PCR primer for cloning of AST promoter (sense primer)

<400> 16
   gcggccgcac gtacagacta agatcgac 28

<210> 17
<211> 33
<212> DNA
<213> Artificial Sequence

<220>
<223> Description of Artificial Sequence:PCR primer for cloning of AST promoter (antisense primer)

<400> 17
   ggccataatg gccatggaga aagtaggtgg caa 33

<210> 18
<211> 39
<212> DNA
<213> Artificial Sequence

<220>
<223> Description of Artificial Sequence:PCR primer for cloning of AST terminator (sense primer)

<400> 18

<210> 19
<211> 26
<212> DNA
<213> Artificial Sequence

<220>
<223> Description of Artificial Sequence:PCR primer for cloning of AST terminator (antisense primer)

<400> 19
   ggtaccctgc agtcgacaaa catgaa 26